# EUROPEAN PATENT APPLICATION

(11) **EP 4 272 700 A1**
(43) Date of publication of application: **08.11.2023**
(21) Application number: 21912993.9
(22) Date of filing: 02.07.2021
(51) Int. Cl.: A61F 2/24, A61F 2/966, A61F 2/97

(54) **IMPLANT DELIVERY SYSTEM**

(30) Priority: 30.12.2020 CN 202023329545 U; 30.12.2020 CN 202011642708
(71) Applicant: Jiangsu Polylive Medtech Co., Ltd., Nantong, Jiangsu 226400 (CN)
(72) Inventor: ZUO, Bin, Shanghai 201702 (CN)
(74) Representative: Argyma
(86) International application number: PCT/CN2021/104351
(87) International publication number: WO 2022/142239

(57) **Abstract**

The present invention disclosed an implant-delivering system, which includes a first handle connected with an outer sheath tube, a second handle connected with an inner pushrod, and an implant loading device arranged at the distal end of the inner pushrod away from the second handle. The outer sheath tube is operated and axially moved relative to the first handle. The inner pushrod is axially moved relative to the second handle. A connection assembly is further arranged between the first handle and the second handle. When the first handle and the second handle are connected through the connection assembly, the inner pushrod is inserted into the outer sheath tube. The implant-delivering system of the present invention can effectively control the speed and distance of releasing implants at each step.

## Description

### BACKGROUND OF THE INVENTION

### Field of the Invention

The present invention relates to a technical field for designing medical devices, particularly to an implant-delivering system.

### Description of the Related Art

Heart failure, referred to the failure of the venous return blood fully discharged from the heart due to the dysfunction of the systolic function and/or diastolic function of the heart, results in blood stasis in the venous system and insufficient blood perfusion in the arterial system to induce cardiac dysfunction syndrome. Heart failure is a serious disease with high morbidity and mortality. According to the location of heart failure, it can be divided into left heart failure, right heart failure and whole heart failure. According to the clinical manifestations of heart failure, it can also be divided into systolic heart failure and diastolic heart failure. The patients with diastolic heart failure (DHF) account for about half of the patients with whole heart failure. There are more than 12 million patients heart failure in China, that is, the incidence rate of heart failure is about 2 to 3%. There are about 6 million patients with diastolic heart failure. The heart failure of the elderly is mostly diastolic heart failure. The number of patients with diastolic heart failure of the elderly accounts for 66.99% of the total number of patients with diastolic heart failure.

In the treatment of heart failure, there are defects in various existing methods. Drug treatment cannot fundamentally remove the cause, and there is still the possibility of recurrence. Cardiac resynchronization therapy (CRT) is ineffective for at least 20% of patients with heart failure. Left ventricular assist device (LVAD) surgery requires extracorporeal circulation, which not only has major trauma and a high incidence of complications, but is also expensive and difficult to obtain. Heart transplantation can fundamentally solve the problem, but the source of donors is very limited and expensive.

The method of interatrial shunt is a new solution for the treatment of heart failure. This solution is to make an opening in the atrial septum between the left atrium and the right atrium of the patient, and implant a shunt device to form a shunt between the left and right atrium. The implantable intra-atrial shunt devices have been successfully used in patients with severe heart failure. By diverting or shunting blood from the left atrium (LA) to the right atrium (RA), the pressure in the left atrium is reduced or prevented from increasing (i.e., left atrial decompresses). This solution can prevent, relieve, or limit symptoms, signs, and syndromes associated with pulmonary congestion, including severe shortness of breath, pulmonary edema, hypoxia, etc.

The implantation of a shunt device requires the use of a delivery system. Generally speaking, the implant is delivered to the interatrial septal position along the catheter sheath of the delivery system. The left-atrium part and the right-atrium part of the implant are sequentially released by the movement of the catheter of the delivery system. Because the implantation of an atrial shunt device needs to release the left-atrium portion part and right-atrium part of the implant sequentially by controlling the relative movement of different parts of the delivery system multiple times, the control difficulty is higher. The control accuracy will directly affect the releasing effect. If the push distance is too long, the implant risks falling into the left atrium. If the push distance is too short, it will affect the release of the right-atrium part. In the conventional technology, only relying on the operator's experience for manual control, it is easy to cause positioning errors, which results in falling implants off and operation failure.

Therefore, there is an urgent need for a delivery system with controllable and easy-to-control release speed and release distance, so as to ensure the accuracy of the implantation position and improve the success rate of the operation.

### SUMMARY OF THE INVENTION

In order to overcome the conventional problems, the present invention provides an implant-delivering system, which includes a first handle connected with an outer sheath tube, a second handle connected with an inner pushrod, and an implant-loading device arranged at the distal end of the inner pushrod away from the second handle. The outer sheath tube is operated and axially moved relative to the first handle. The inner pushrod is axially moved relative to the second handle. A connection assembly is further arranged between the first handle and the second handle. When the first handle and the second handle are connected through the connection assembly, the inner pushrod is inserted into the outer sheath tube.

In some embodiments, the proximal end of the outer sheath tube and the proximal end of the inner pushrod are respectively axially and adjustably installed on the first handle and the second handle through a finely-adjusting assembly. In some embodiments, the finely-adjusting assembly includes:
an adjustment rod with one end thereof axially and movably installed in the first handle/second handle, and another end of the adjustment rod is provided with an adjustment portion; the proximal end of the outer sheath tube/the inner pushrod is coaxially connected with the adjustment rod;
an adjustment member, installed on the first handle/second handle and correspondingly connected with the adjustment portion, driving the adjustment member to axially move the adjustment rod.

In some embodiments, the adjustment portion is implemented with a threaded rod, the adjustment member is implemented with a threaded knob, the threaded knob sleeves the threaded rod, and the threaded knob is threaded with the threaded rod. The threaded knob, which is axially limited and circumferentially rotatable, is installed on the first handle/second handle.

In some embodiments, the adjustment rod is provided with at least one guide protrusion. The guide protrusion is parallel to the axial direction of the adjustment rod. The corresponding position of the first handle/second handle is provided with a guide groove. The guide protrusion is located in the guide groove. The guide protrusion moves along the guide groove.

In some embodiments, locking devices are arranged between the first handle and the adjustment rod and between the second handle and the adjustment rod, respectively. The locking devices are used to limit the axial movement of the adjustment rod relative to the first handle and the second handle.

In some embodiments, the locking device includes a locking ring. The locking ring annularly sleeves the adjustment rod. A locking-ring elastic member is arranged between one side of the locking ring and the inner wall of the first handle/second handle. Another side of the locking ring is provided with a button protruding from the first handle/second handle.

A locking portion is arranged on the inner ring of the locking ring and the relative location of the locking-ring elastic member. The locking portion abuts against the adjustment rod to achieve locking by an action of the locking-ring elastic member. When the button is pressed to move the locking ring, the locking portion disengages from the adjustment rod to achieve unlocking.

In some embodiments, when the adjustment portion on the adjustment rod is a threaded rod, the locking portion is a threaded portion matched with the threaded rod. The threaded portion cooperates with the threaded rod to achieve locking.

In some embodiments, the connection assembly includes a first hook-like structure on the first handle, a second hook-like structure on the second handle, and an unlocking structure on the first handle or the second handle.

The first handle is coaxially connected with the second handle. The first hook-like structure and the second hook-like structure are buckled together to achieve fixation. When pushing the unlocking structure, the unlocking structure pushes the first hook-like structure or the second hook-like structure, so that the first hook-like structure is separated from the second hook-like structure to achieve unlocking.

In some embodiments, the unlocking structure includes:
a slider, the casing of the second handle is provided with a chute, and the slider is located in the chute;
a first incline structure located in the second handle and connected with the slider;
a second incline structure arranged on the second hook-like structure;
after the first hook-like structure is connected with the second hook-like structure, the first incline structure abuts against the second incline structure; when sliding the slider, the first incline structure slides along the second incline structure, thereby pushing the second hook-like structure to move radially along the second handle and to separate from the first hook-like structure.

In some embodiments, a slider elastic member is arranged between the slider and the chute. When the slider moves along the chute to unlock, the slider elastic member deforms to store energy, and the slider resets by an action of the slider elastic member after unlocking.

In some embodiments, the implant-loading device includes a loading catheter arranged at the distal end of the inner pushrod. The distal end of the inner pushrod axially and movably passes through the loading catheter. A fixing member for fixing an implant is further arranged at the distal end of the inner pushrod.

In some embodiments, a reinforcing tube is also arranged at the proximal end of the inner pushrod close to the second handle.

Compared with the conventional technology, the present invention has the following advantages and positive effects due to the foregoing technical implementations:
In the implant-delivering system of the present invention, the outer sheath tube and the inner pushrod are independently arranged, and the movement of the outer sheath tube and the inner pushrod can be controlled independently, so that they can perform the functions of the catheter sheath and the pushrod respectively. The outer sheath tube and the inner pushrod can also be connected through the first handle, the second handle, and the connection assembly. The outer sheath tube and the inner pushrod cooperate with each other to stably and precisely deliver the implant, which greatly broadens the range of application. Further, when the first handle is used in conjunction with the second handle, the implant is first loaded into the implant-loading device at the distal end of the inner pushrod, the implant-loading device with the implant and the inner pushrod are inserted into the outer sheath tube, and the inner pushrod pushes the implant from the implant-loading device into the outer sheath tube. The inner pushrod pushes the implant forward to the distal end along the outer sheath tube until the first handle and the second handle are connected together to form a complete handle. Then,operate the inner pushrod to make it axially move towards to the distal end relative to the second handle. At this time, the outer sheath tube is relatively fixed, and a part of the implant is gradually released from the distal end of the outer sheath tube. Then, adjust the outer sheath tube and make it axially move towards to the proximal end relative to the first handle. At this time, the inner pushrod is relatively fixed, so that the rest of the implant is gradually released from the distal end of the outer sheath tube. This implant-pushing method can effectively control the speed and distance of releasing implants at each step. Compared with the conventional method for directly pushing the implant by hand, the controllability for releasing implants is implemented, which effectively prevents from the releasing accident caused by the control error due to the push of the inner pushrod or the retreat of the outer sheath during the release of the implant.

### BRIEF DESCRIPTION OF THE DRAWINGS

The foregoing and other features and advantages of the present invention can be more clearly understood through the following detailed description in conjunction with the accompanying drawings, wherein:
Fig. 1 is a diagram schematically illustrating an implant-delivering system according to an embodiment of the present invention;
Fig.2 is a diagram schematically illustrating an inner pushrod loaded into an outer sheath tube according to an embodiment of the present invention;
Fig.3 is a diagram schematically illustrating an inner pushrod separating from an outer sheath tube according to an embodiment of the present invention;
Fig.4 is a cross-sectional view of an implant-delivering system according to an embodiment of the present invention;
Fig.5 is a partial cross-sectional view of a finely-adjusting assembly and a locking device according to an embodiment of the present invention;
Fig.6 is a diagram schematically illustrating an adjustment rod in locking according to an embodiment of the present invention;
Fig.7 is a diagram schematically illustrating an adjustment rod after unlocking according to an embodiment of the present invention;
Fig. 8 is a diagram schematically illustrating a connection assembly according to an embodiment of the present invention;
Fig.9 is a diagram schematically illustrating an implant-loading device according to an embodiment of the present invention;
Fig. 10 is a diagram schematically illustrating a fixing member according to an embodiment of the present invention;
Fig. 11 is a diagram schematically illustrating a cardiac shunt device according to an embodiment of the present invention; and
Fig. 12 is a diagram schematically illustrating a cardiac shunt device installed on the interatrial septum of the present invention.

### DETAILED DESCRIPTION OF THE INVENTION

The present invention will be described in more detail hereinafter with reference to the accompanying drawings showing embodiments of the invention. However, the present invention may be implemented in many different forms and should not be limited to the embodiments set forth herein. Rather, these embodiments are provided so that this disclosure will be thorough and complete, and that those skilled in the art will fully appreciate the scope of the present invention. In these drawings, the size and relative sizes of the hierarchical regions may be exaggerated for clarity.

It should be noted that all directional indications (such as up, down, left, right, front, back, etc.) in the embodiments of the present invention are only used to explain the relationship between the components in a certain posture (as shown in the drawing), such as relative positional relationships, movement states, etc. If the specific posture changes, the directional indication will also change accordingly.

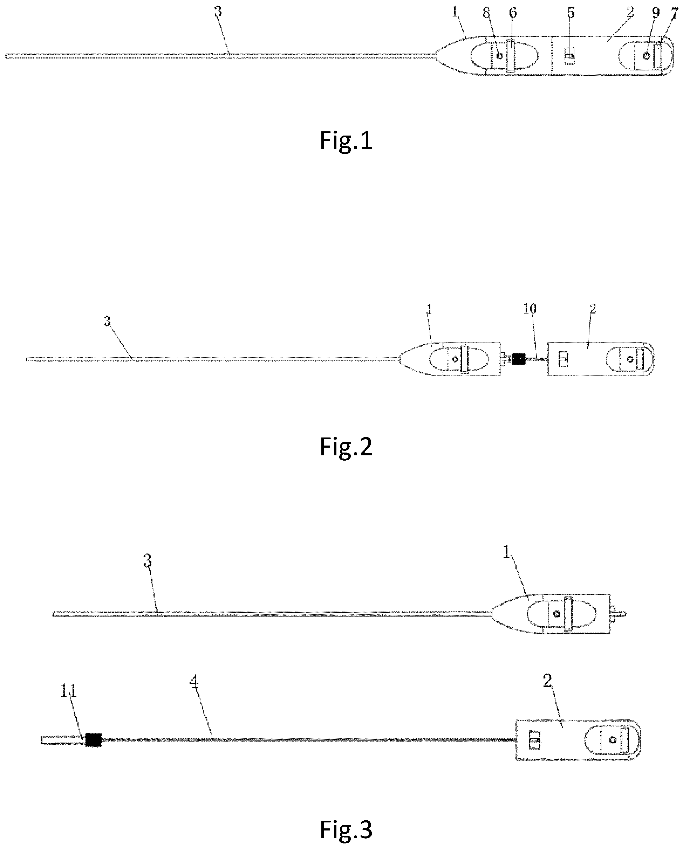

Refer to Figs. 1-10. The present invention provides an implant-delivering system that includes a first handle 1 connected with an outer sheath tube 3, a second handle 2 connected with an inner pushrod 4, and an implant-loading device arranged at the distal end of the inner pushrod 4 away from the second handle 2. The outer sheath tube 3 may be operated and axially moved relative to the first handle 1. The inner pushrod 4 may be axially moved relative to the second handle 2. A connection assembly 5 is further arranged between the first handle 1 and the second handle 2. The inner pushrod 4 is inserted into the outer sheath tube 3 when the first handle 1 and the second handle 2 are connected through the connection assembly 5.

Wherein, one end close to an operator is defined as a proximal end, and one end far away from the operator is defined as a distal end. That is to say, in the present invention, one end close to the first handle 1 and the second handle 2 is defined as the proximal end, and another end opposite to the end close to the first handle 1 and the second handle 2 is defined as the distal end.

In the implant-delivering system of the present invention, the outer sheath tube 3 and the inner pushrod 4 are independently arranged, and the movement of the outer sheath tube and the inner pushrod can be controlled independently, so that they can perform the functions of the catheter sheath and the pushrod respectively. The outer sheath tube 3 and the inner pushrod 4 can also be connected through the first handle, the second handle, and the connection assembly. The outer sheath tube and the inner pushrod cooperate with each other to stably and precisely deliver the implant, which greatly broadens the range of application.

Further, when the first handle 1 is used in conjunction with the second handle 2, the implant is first loaded into the implant-loading device 11 at the distal end of the inner pushrod 4, the implant-loading device with the implant and the inner pushrod are inserted into the outer sheath tube 3, and the inner pushrod pushes the implant from the implant-loading device into the outer sheath tube. The inner pushrod pushes the implant forward to the distal end along the outer sheath tube until the first handle 1 and the second handle 2 are connected together to form a complete handle. Then, the inner pushrod to make it axially move towards to the distal end relative to the second handle 2. At this time, the outer sheath tube is relatively fixed, and a part of the pushed implant is gradually released from the distal end of the outer sheath tube 3. Then, adjust the outer sheath tube 3 and make it axially move towards to the proximal end relative to the first handle 1. At this time, the inner pushrod is relatively fixed, so that the rest of the implant is gradually released from the distal end of the outer sheath tube. This implant-pushing method can effectively control the speed and distance of releasing implants at each step. Compared with the conventional method for directly pushing the implant by hand, the controllability for releasing implants is implemented, which effectively prevents from the releasing accident caused by the control error due to the push of the inner pushrod during the release of the implant.

The implant-delivering system of the present invention is applied to the delivery of various types of implants, but the present invention is not limited thereto. It is especially suitable for the delivery of cardiac shunt devices. The following uses the application of cardiac bypass devices as an example for specific description.

In the embodiment, the proximal end of the outer sheath tube 3 is axially and adjustably installed on the first handle 1 through a first finely-adjusting assembly.

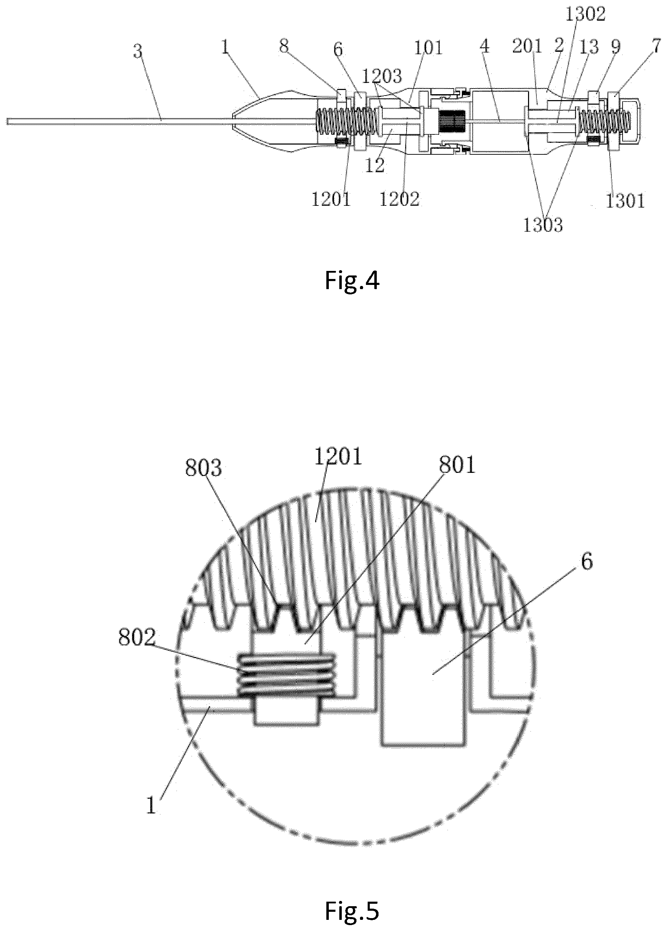

Specifically, as illustrated in Fig.4, the first finely-adjusting assembly includes a first adjustment rod 12 and a first adjustment member 6. The proximal end of the first adjustment rod 12 is axially and movably installed in the first handle 1, and the distal end of the first adjustment rod 12 is provided with a first adjustment portion 1201. The proximal end of the outer sheath tube 3 is coaxially connected with the first adjustment rod 12. The first adjustment rod 12 is axially provided with a through hole communicating with the outer sheath tube 3. The first adjustment member 6 is installed on the first handle 1 and correspondingly connected with the first adjustment portion 1201 to drive the first adjustment member 6 to axially move the first adjustment rod 12, thereby causing the adjustment of the axial movement of the outer sheath tube 3. In the embodiment, the first finely-adjusting assembly precisely controls the moving speed and moving distance of the outer sheath tube 3 that moves axially relative to the first handle 1, so that the releasing speed of the implant can be controlled to prevent displacement caused by rapidly releasing the implant. In addition, in the embodiment, the first adjustment rod 12 and the sheath seat of the outer sheath tube 3 are combined into one, which not only satisfies the basic function of the sheath seat, but also implements the finely-adjusting movement of the outer sheath tube, thereby greatly saving the space of the first handle 1.

Wherein, the proximal end 1203 of the first adjustment rod 12 is axially and movably installed on the first handle 1. The port of the first adjustment rod 12 is provided with a hemostatic valve, so that the hemostatic valve can be used as a sheath seat alone.

Wherein, one or more first guide protrusions 1202 are also arranged on the outer wall of the first adjustment rod 12. The inner wall of the first handle 1 corresponding to each first guide protrusion 1202 is provided with matching first guide grooves. The first guide protrusion 1202 and the first guide groove are arranged parallel to the axial extension direction of the outer sheath tube 3. The first guide protrusion 1202, arranged in the first guide groove, can move along the first guide groove. In the embodiment, the axial linear movement of the first adjustment rod 12 and the outer sheath tube 3 is guaranteed using the cooperative arrangement of the first guide protrusion 1202 and the first guide groove. At the same time, the function of radial positioning is implemented. Certainly, in other embodiments, the guide structures of the first adjustment rod 12 and the first handle 1 are not limited to the foregoing structures. For example, the first handle 1 may be provided with a guide protrusion and the first adjustment rod 12 may be provided with a guide groove, but the present invention is not limited thereto. The guide structures of the first adjustment rod 12 and the first handle 1 can be modified according to specific conditions.

Further, the inner wall of the first handle 1 is provided with a first protruding ring 101. The first guide groove is arranged on the inner wall of the first protruding ring 101. The first adjustment rod 12 passes through the first protruding ring 101. The first guide protrusion 1202 is located in the first guide groove. The first adjustment rod 12 is also provided with two first positioning rings 1203. The two first positioning rings 1203 are respectively located at the both ends of the first guide protrusion 1202 and located on the both side of the axial direction of the first protruding ring 101. The first positioning ring 1203 is used to limit the distance of the axial movement of the first adjustment rod 12 relative to the first handle 1.

Wherein, the first adjustment portion 1201 is implemented with a threaded rod, and the first adjustment member 6 is implemented with a threaded knob. The threaded knob sleeves the threaded rod and the threaded knob is threaded with the threaded rod. The threaded knob, which is axially limited and circumferentially rotatable, is installed on the first handle 1. Turning the threaded knob clockwise or counterclockwise can control the forward and backward movement of the outer sheath tube. Further, the first handle 1 is provided with a depression so that a part of the threaded knob at least protrudes from the first handle 1, so as to facilitate the operator to control the threaded knob. Furthermore, the surface of the threaded knob is provided with a concave-convex structure to increase contact friction.

Certainly, in other embodiments, the implementation of the first adjustment portion 1201 and the first adjustment member 6 is not limited to the cooperative implementation of the threaded rod and the threaded knob. The first adjustment portion 1201 and the first adjustment member 6 may also be implemented with other finely-adjusting structures, such as the cooperative implementation of a rack and a gear, the cooperative implementation of slide-rail type structures, the cooperative implementation of ratchet type structures, etc. As long as it can make the rotation and moving process slow and controllable, the implementation of the first adjustment portion 1201 and the first adjustment member 6 is not limited. The implementation of the first adjustment portion 1201 and the first adjustment member 6 can be modified according to specific conditions.

In the embodiment, a first locking device 8 is further arranged between the first handle 1 and the first adjustment rod 12. The first locking device 8 is used to limit the axial movement of the first adjustment rod 12 relative to the first handle 1. Under normal conditions, the first locking device 8 implements the fixed arrangement of the outer sheath tube 3 relative to the first handle 1. When the movement of the outer sheath tube 3 needs to be adjusted, the first locking device 8 is unlocked. The arrangement of the first locking device 8 can prevent from the unnecessary operation of the handle and the movement of the outer sheath tube 3.

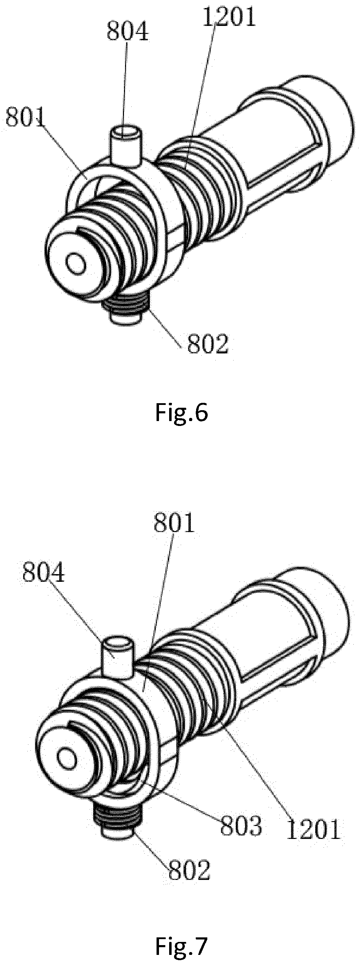

Specifically, refer to Figs.5-7. The first locking device 8 includes a first locking ring 801. The first locking ring 801 sleeves one ring of the threaded rod of the first adjustment rod 12. A first locking-ring elastic member 802 is arranged between one side of the first locking ring 801 and the inner wall of the first handle 1, and another side of the first locking ring 801 is provided with a button 804 protruding from the first handle 1. A first locking portion 803 is arranged on the inner ring of the first locking ring 801 and the relative location of the first locking-ring elastic member 802. The first locking portion 803 abuts against the threaded rod to achieve locking by an action of the first locking-ring elastic member 802, as illustrated in Fig.6. When the first button 804 is pressed to move the first locking ring 801, the first locking portion 803 disengages from the threaded rod to achieve unlocking, as illustrated in Fig.7.

Further, the first locking-ring elastic member 802 may specifically be implemented with a spring or the like, but the present invention is not limited thereto.

Further, the first locking portion 803 is a threaded portion matched with the threaded rod. The threaded portion cooperates with the threaded rod to achieve locking. Certainly, in other embodiments, the first locking portion 803 can also be a protrusion or the like that is fastened with the threaded portion of the threaded rod, but the present invention is not limited thereto. The implementation of the first locking portion 803 can be modified according to specific conditions.

Certainly, in other embodiments, the first locking device 8 is not limited to the foregoing implementations. The implementation of the first locking device 8 can be modified according to specific conditions. In some embodiments, when the first finely-adjusting assembly has a self-locking function, the arrangement of the first locking device 8 can be omitted. The present invention is not limited to the arrangement of the first locking device 8. The arrangement of the first locking device 8 can be adjusted according to specific conditions.

In the embodiment, the proximal end of the inner pushrod 4 is axially and adjustably installed on the second handle 2 using a second finely-adjusting assembly.

Specifically, as illustrated in Fig.4, the second finely-adjusting assembly includes a second adjustment rod 13 and a second adjustment member 7. The distal end of the second adjustment rod 13 is axially and movably installed in the second handle 2, and the proximal end of the second adjustment rod 13 is provided with a second adjustment portion 1301. The proximal end of the inner pushrod 4 is coaxially connected with the second adjustment rod 13. The second adjustment member 7 is installed on the second handle 2 and correspondingly connected with the second adjustment portion 1301 to drive the second adjustment member 7 to axially move the second adjustment rod 13, thereby causing the adjustment of the axial movement of the inner pushrod 4. In the embodiment, the second finely-adjusting assembly precisely controls the moving speed and moving distance of the inner pushrod 4 that moves axially relative to the second handle 2, thereby guaranteeing the safety and precision during the releasing process.

Wherein, the outer wall of the second adjusting rod 13 is further provided with one or more second guide protrusions 1302. The inner wall of the second handle 2 corresponding to each second guide protrusion 1302 is provided with matching second guide grooves. The second guide protrusion 1302 and the second guide groove are parallel to the axial extension direction of the inner pushrod 4. The second guide protrusion 1302, located in the second guide groove, can move along the second guide groove. In the embodiment, using the cooperative arrangement of the second guide groove and the second guide protrusion 1302, the axial linear movement of the second adjustment rod 13 and the inner pushrod 4 is guaranteed to implement the function of radial positioning. Certainly, in other embodiments, the guide structures of the second adjustment rod 13 and the second handle 2 are not limited to the foregoing structures. For example, they can also be a guide protrusion arranged on the second handle 2 and a guide groove on the second adjustment rod 13, but the present invention is not limited thereto. The guide structures of the second adjustment rod 13 and the second handle 2 can be modified according to specific conditions.

Further, the inner wall of the second handle 2 is provided with a second protruding ring 201. The second guide groove is arranged on the inner wall of the second protruding ring 201. The second adjustment rod 13 passes through the second protruding ring 201. The second guide protrusion 1302 is located in the second guide groove. The second adjustment rod 13 is also provided with two second positioning rings 1303. The two second positioning rings 1303 are respectively located at the both ends of the second guide protrusion 1302 and located on the both sides of the axial direction of the second protruding ring 201. The second positioning ring 1303 is used to limit the distance of the axial movement of the second adjustment rod 13 relative to the second handle 2.

Wherein, the second adjustment portion 1301 is implemented with a threaded rod, and the second adjustment member 7 is implemented with a threaded knob. The threaded knob sleeves the threaded rod, and the threaded knob is threaded with the threaded rod. The threaded knob, which is axially limited and circumferentially rotatable, is installed on the second handle 2. Turning the threaded knob clockwise or counterclockwise can control the forward and backward movement of the inner pushrod 4. Further, the second handle 2 is provided with a depression so that a part of the threaded knob at least protrudes from the second handle 2, so as to facilitate the operator to control the threaded knob. Furthermore, the surface of the threaded knob is provided with a concave-convex structure to increase contact friction.

Certainly, in other embodiments, the implementation of the second adjustment portion 1301 and the second adjustment member 7 is not limited to the cooperative implementation of the threaded rod and the threaded knob. The second adjustment portion 1301 and the second adjustment member 7 may also be implemented with other finely-adjusting structures, such as the cooperative implementation of a rack and a gear, the cooperative implementation of slide-rail type structures, the cooperative implementation of ratchet type structures, etc. As long as it can make the rotation and moving process slow and controllable, the implementation of the second adjustment portion 1301 and the second adjustment member 6 is not limited. The implementation of the second adjustment portion 1301 and the second adjustment member 6 can be modified according to specific conditions.

In the embodiment, a reinforcing tube 10 is arranged on the proximal end of the inner pushrod 4 connected to the second handle 2, and the reinforcing tube 10 is used to increase the rigidity of the proximal end of the inner pushrod 4.

Wherein, the material of the reinforcing tube 10 can be selected from stainless steel metal materials with good biocompatibility, high polymer materials with high hardness, etc., which can prevent the inner pushrod 4 from being bent due to its own insufficient rigidity when pushing, so that the inner pushrod 4 continues to deliver the implant.

Wherein, the reinforcing tube 10 can be connected to the inner pushrod 4 by means of splicing or sleeving, etc., but the present invention is not limited thereto. The method of connecting the reinforcing tube 10 with the inner pushrod 4 can be adjusted according to specific conditions.

In the embodiment, a second locking device 9 is further arranged between the second handle 2 and the second adjustment rod 13. The second locking device 9 is used to limit the axial movement of the second adjustment rod 13 relative to the second handle 2. Under normal conditions, the second locking device 9 implements the fixed arrangement of the inner pushrod 4 relative to the second handle 2. When the movement of the inner pushrod 4 needs to be adjusted, the second locking device 9 is unlocked. The arrangement of the second locking device 9 can prevent from the unnecessary operation of the handle and the movement of the inner pushrod.

Specifically, the second locking device 9 includes a second locking ring. The second locking ring sleeves one ring of the threaded rod of the second adjustment rod 13. A second locking-ring elastic member is arranged between one side of the second locking ring and the inner wall of the second handle 2, and another side of the second locking ring is provided with a button protruding from the second handle 2. A second locking portion is arranged on the inner ring of the second locking ring and the relative location of the second locking-ring elastic member. The second locking portion is pressed against the threaded rod to achieve locking by an action of the second locking-ring elastic member. When the second button is pressed to move the second locking ring, the second locking portion disengages from the threaded rod to achieve unlocking. The specific structure of the second locking device 9 can be similar to that of the first locking device 8.

Further, the second locking portion is a threaded portion matched with the threaded rod. The threaded portion cooperates with the threaded rod to achieve locking. Certainly, in other embodiments, the second locking portion can also be a protrusion or the like that is fastened with the threaded portion of the threaded rod, but the present invention is not limited thereto. The implementation of the second locking portion can be modified according to specific conditions.

Certainly, in other embodiments, the second locking device 9 is not limited to the foregoing implementations. The implementation of the second locking device 9 can be modified according to specific conditions. In some embodiments, when the second finely-adjusting assembly has a self-locking function, the arrangement of the second locking device 9 can be omitted. The present invention is not limited to the arrangement of the second locking device 9. The arrangement of the second locking device 9 can be adjusted according to specific conditions.

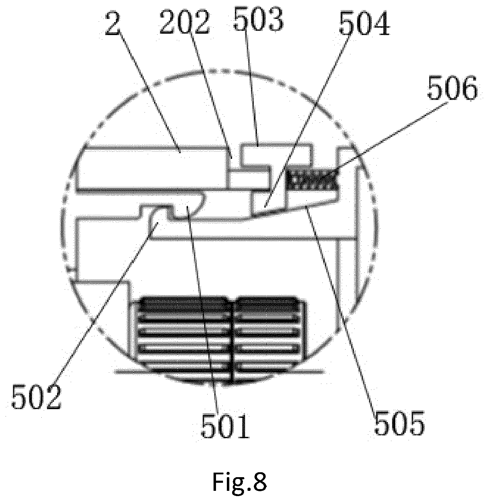

In the embodiment, referring to Fig.4 and Fig.8, the connection assembly includes a first hook-like structure 501 on the first handle 1, a second hook-like structure 502 on the second handle 2, and an unlocking structure on the first handle 1 or the second handle 2. The first handle 1 and the second handle 2 are coaxially connected with each other. The first hook-like structure 501 and the second hook-like structure 502 are hooked together to implement a locking connection. When backward sliding the unlocking structure, the unlocking structure pushes the first hook-like structure 501 or the second hook-like structure 502, so that the first hook-like structure 501 is separated from the second hook-like structure 502 to achieve unlocking.

Further, as illustrated in Fig.8, the unlocking structure includes a slider 503, a first incline structure 504, and a second incline structure 505. The side wall of the second handle 2 is provided with a chute 202. The slider 503, located in the chute 202, can move along the chute 202. The moving direction of the slider 503 is parallel to the axial directions of the inner pushrod 4 and the outer sheath tube 3. The first incline structure 504 is located in the second handle 2 and connected with the slider 503 through a connecting portion. The second hook-like structure 502 is located below the chute 202. The second incline structure 505 is arranged on the second hook-like structure 502 and arranged opposite to the first incline structure 504. When the first hook-like structure 501 and the second hook-like structure 502 are connected together, the first incline structure 504 abuts against the second incline structure 505. When sliding the slider 503, the first incline structure 504 slides along the second incline structure 505 to push and radially move the second hook-like structure 502 along the second handle 2, so that the second hook-like structure 502 is separated from the first hook-like structure 501 to achieve unlocking.

Further, a slider elastic member 506 is arranged between the slider 503 and the chute 202. When the slider 503 moves along the chute 202 to unlock, the slider elastic member 506 deforms to store energy. After the unlocking is completed, the slider resets under the action of the slider elastic member 506. Wherein, the elastic member 506 can be specifically implemented with a spring, but the present invention is not limited thereto.

The connection assembly of the embodiment is implemented with an elastic buckle, which has the advantages of simple structure and convenient operation. Certainly, other connection structures can also be used in other embodiments, but the present invention is not limited thereto.

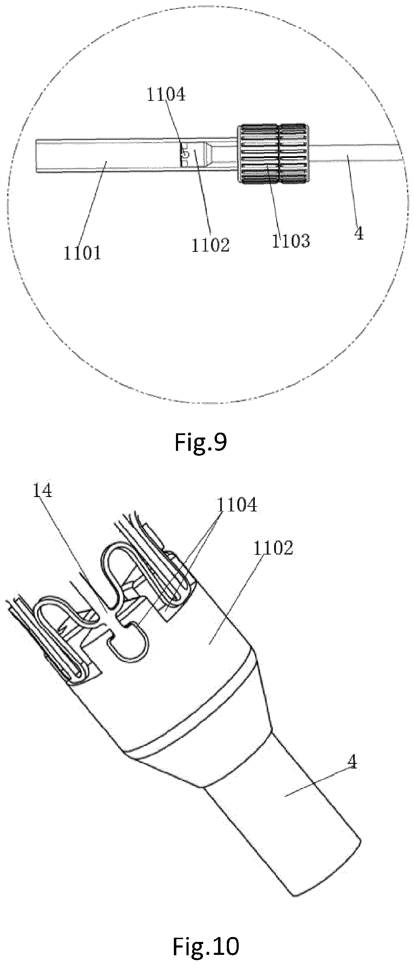

In the embodiment, referring to Figs. 9-10, the implant-loading device 11 includes a loading catheter 1101 arranged at the distal end of the inner pushrod 4. Specifically, the loading catheter 1101 is installed at the distal end of the inner pushrod 4 through a loading catheter socket 1103. The inner pushrod 4 can move axially relative to the loading catheter 1101 and the loading catheter socket 1103. The distal end of the inner pushrod 4 is axially and movably installed in the loading catheter 1101. A fixing member 1102 for fixing the implant is further arranged at the distal end of the inner pushrod 4.

Wherein, the fixing member 1102 is provided with a recess structure 1104 for fixing the implant 14, as illustrated in Fig.10. The implant, sleeving the end of the fixing member 1102, is partially located in the recess structure 1104. Certainly, the specific structure of the fixing member 1102 is not limited to the foregoing structure. The specific structure of the fixing member 1102 can be modified based on the structure of the implant, but the present invention is not limited thereto.

Wherein, the material of the fixing member 1102 can be selected from stainless steel or other metals or polymer materials with suitable hardness and good biocompatibility, but the present invention is not limited thereto. The material of the fixing member 1102 can be selected according to specific conditions.

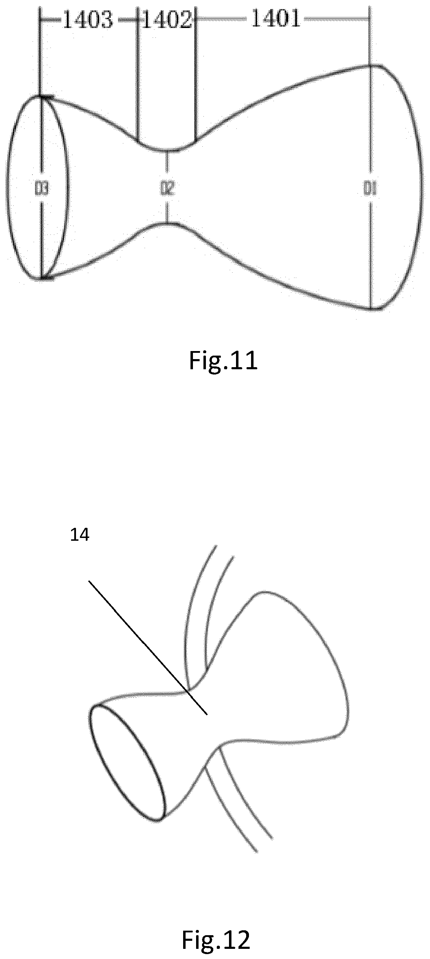

An example of the application of the present invention to the delivery of a cardiac shunt device is introduced as follows. As illustrated in Figs. 11-12, the cardiac shunt device is divided into a first area 1401, a second area 1402, and a third area 1403. The cardiac shunt device 14 may be implanted by the following methods. A puncture is formed to pass through the interatrial septum and the cardiac shunt device 14 is then percutaneously inserted into the puncture such that the cardiac shunt device 14 passes through the puncture. Thus, the first area 1401 extends into the patient's left atrium, the second area 1402 is embedded in the puncture, and the third area 1403 extends into the patient's right atrium. Preferably, the third area 1403 extends at least 5 mm into the patient's right atrium, so that the outlet of the third area 1403 is located outside the natural circulation flowing path of blood entering the patient's right atrium from the inferior vena cava, which prevents the thrombus entrained in the flow of the inferior vena cava from being directed into the outlet of the third region 1403.

The release of the implant 14 is divided into release of the left atrial stent and release of the right atrial stent. The specific operation is introduced as follows:
1. Load and crimp the implant 14 into the loading catheter 1101. Specifically, a lug structure on the implant 14 is correspondingly connected with the recess structure 1104 on the fixing member 1102, such that the implant is crimped into the loading catheter 1101.
2. Push the loading catheter 1101 with the implant into the outer sheath tube 3, and then push the inner pushrod 4. The fixing member 1102 is used to push the implant 14 from the loading catheter into the outer sheath tube 3. The loading catheter 1101 stays at the proximal end of the outer sheath tube 3.
3. Continue to push the second handle 2. The inner pushrod 4 pushes the implant 14 so that the implant 14 advances along the inner wall of the outer sheath tube 3 until the first handle 1 and the second handle 2 are combined, locked by the connecting assembly, and mutually secured to form a complete handle. After combining the handles, there is a distance of 3-5mm between the implant 14 and the head end exposed from the distal end of the outer sheath tube 3 (i.e., the distance tolerance is compensated according to the assembly error).
4. Press down the button of the second locking device 9 and keep it pressed down, and then turn the threaded knob on the second handle 2 to push the inner pushrod 4 to continue to move distally. The left atrium part of the implant 14 is slowly released from the head end of the outer sheath tube 3. When it is released to the waist (1402 area) of the implant 14, the operation of the threaded knob is stopped and the second locking device 9 is released, so that the inner pushrod 4 is restored and fixed relative to the second handle 2.
5. After the left atrium part is completely released, the entire delivery system retreats, so that the delivery system and the implant 14 retreat until the waist of the implant 14 is just stuck on the interatrial septum.
6. Press down the button 804 of the first locking device 8 and keep it pressed down. Turn the threaded knob on the first handle, such that the outer sheath tube 3 retreats. Thus, the right atrium part (i.e., 1403 area) of the implant 14 is slowly released until the hanging ear is fully exposed and the implant 14 is separated from the fixing member to implement the complete release. Finally, the button 804 of the first locking device 8 is released.
7. After the release is completed, the overall delivery system retreats from the body.

The embodiments described above are only to exemplify the present invention but not to limit the scope of the present invention. Therefore, any equivalent modification or variation according to the shapes, structures, features, or spirit disclosed by the present invention is to be also included within the scope of the present invention.

## Claims

1. An implant-delivering system comprising a first handle connected with an outer sheath tube, a second handle connected with an inner pushrod, and an implant-loading device arranged at a distal end of the inner pushrod away from the second handle, the outer sheath tube is operated and axially moved relative to the first handle, and the inner pushrod is axially moved relative to the second handle; a connection assembly further arranged between the first handle and the second handle, when the first handle and the second handle are connected through the connection assembly, the inner pushrod is inserted into the outer sheath tube.

2. The implant-delivering system according to claim 1, wherein a proximal end of the outer sheath tube and a proximal end of the inner pushrod are respectively axially and adjustably installed on the first handle and the second handle through a finely-adjusting assembly; preferably, the finely-adjusting assembly comprises:
an adjustment rod with one end thereof axially and movably installed in the first handle/second handle, and another end of the adjustment rod is provided with an adjustment portion; a proximal end of the outer sheath tube/the inner pushrod is coaxially connected with the adjustment rod;
an adjustment member, installed on the first handle/second handle and correspondingly connected with the adjustment portion, driving the adjustment member to axially move the adjustment rod.

3. The implant-delivering system according to claim 2, wherein the adjustment portion is implemented with a threaded rod, the adjustment member is implemented with a threaded knob, the threaded knob sleeves the threaded rod, the threaded knob is threaded with the threaded rod, and the threaded knob, which is axially limited and circumferentially rotatable, is installed on the first handle/second handle.

4. The implant-delivering system according to claim 2, wherein the adjustment rod is provided with at least one guide protrusion, the guide protrusion is parallel to an axial direction of the adjustment rod, a corresponding position of the first handle/second handle is provided with a guide groove, the guide protrusion is located in the guide groove, and the guide protrusion moves along the guide groove.

5. The implant-delivering system according to claims 2 or 3, wherein locking devices are arranged between the first handle and the adjustment rod and between the second handle and the adjustment rod, respectively, and the locking devices are used to limit axial movement of the adjustment rod relative to the first handle and the second handle.

6. The implant-delivering system according to claim 5, wherein the locking device comprises a locking ring, the locking ring annularly sleeves the adjustment rod, a locking-ring elastic member is arranged between one side of the locking ring and an inner wall of the first handle/second handle, and another side of the locking ring is provided with a button protruding from the first handle/second handle;
a locking portion is arranged on an inner ring of the locking ring and a relative location of the locking-ring elastic member, the locking portion abuts against the adjustment rod to achieve locking by an action of the locking-ring elastic member, and when the button is pressed to move the locking ring, the locking portion disengages from the adjustment rod to achieve unlocking.

7. The implant-delivering system according to claim 6, wherein when the adjustment portion on the adjustment rod is a threaded rod, the locking portion is a threaded portion matched with the threaded rod, and the threaded portion cooperates with the threaded rod to achieve locking.

8. The implant-delivering system according to claim 1, wherein the connection assembly comprises a first hook-like structure on the first handle, a second hook-like structure on the second handle, and an unlocking structure on the first handle or the second handle;
the first handle is coaxially connected with the second handle, and the first hook-like structure and the second hook-like structure are buckled together to achieve fixation; when pushing the unlocking structure, the unlocking structure pushes the first hook-like structure or the second hook-like structure, so that the first hook-like structure is separated from the second hook-like structure to achieve unlocking.

9. The implant-delivering system according to claim 8, wherein the unlocking structure comprises:
a slider, a casing of the second handle is provided with a chute, and the slider is located in the chute;
a first incline structure located in the second handle and connected with the slider;
a second incline structure arranged on the second hook-like structure;
after the first hook-like structure is connected with the second hook-like structure, the first incline structure abuts against the second incline structure; when sliding the slider, the first incline structure slides along the second incline structure, thereby pushing the second hook-like structure to move radially along the second handle and to separate from the first hook-like structure.

10. The implant-delivering system according to claim 9, wherein a slider elastic member is arranged between the slider and the chute, when the slider moves along the chute to unlock, the slider elastic member deforms to store energy, and the slider resets by an action of the slider elastic member after unlocking.

11. The implant-delivering system according to claim 1, wherein the implant-loading device comprises a loading catheter arranged at a distal end of the inner pushrod, and a distal end of the inner pushrod axially and movably passes through the loading catheter; a fixing member for fixing an implant is further arranged at a distal end of the inner pushrod, preferably, a reinforcing tube is further arranged at a proximal end of the inner pushrod close to the second handle.
